# EUROPÄISCHE PATENTSCHRIFT

(11) **EP 2 038 045 B1**
(45) Veröffentlichungstag und Bekanntmachung des Hinweises auf die Patenterteilung: **21.04.2010**
(21) Anmeldenummer: 07764768.3
(22) Anmeldetag: 21.06.2007
(51) Int. Cl.: B01D 53/14, B01D 53/52, B01D 53/58, B01D 53/78, C07C 7/11

(54) **VERFAHREN ZUR REINIGUNG VON BIOGAS EINER BIOGASANLAGE SOWIE GASREINIGUNGSANLAGE**
METHOD FOR PURIFYING BIOGAS FROM A BIOGAS INSTALLATION, AND GAS PURIFICATION SYSTEM
PROCÉDÉ DE PURIFICATION DE BIOGAZ PRODUIT PAR UNE INSTALLATION DE PRODUCTION DE BIOGAZ ET SYSTÈME DE PURIFICATION DE BIOGAZ

(30) Priorität: 30.06.2006 DE 102006030773
(43) Veröffentlichungstag der Anmeldung: 25.03.2009
(73) Patentinhaber: Biomethan N.e.w. Gmbh, 80686 Müchen (DE)
(72) Erfinder: APFELBÖCK, Markus, 94405 Landau a.d. Isar (DE)
(74) Vertreter: Liebl, Thomas
(86) Internationale Anmeldenummer: PCT/EP2007/005482
(87) Internationale Veröffentlichungsnummer: WO 2008/000388

(56) Entgegenhaltungen:
- EP-A- 0 180 670
- DE-A1- 10 119 991
- DE-A1- 10 356 276
- DE-B3-102005 051 952
- US-B1- 6 365 099

## Beschreibung

Die Erfindung betrifft ein Verfahren zur Reinigung von Biogas einer Biogasanlage nach dem Oberbegriff des Anspruchs 1 sowie eine Gasreinigungsanlage zur Reinigung von Biogas einer Biogasanlage nach dem Oberbegriff des Anspruchs 8.

Das aus dem Fermenter einer Biogasanlage gewonnene Biogas beinhaltet neben dem gewünschten Methan (CH₄) auch Kohlendioxid (CO₂), Schwefelwasserstoff (H₂S), Ammoniak (NH₃) und gesättigten Wasserdampf. Diese eben drei genannten Biogas-Bestandteile Kohlendioxid, Schwefelwasserstoff und Ammoniak beeinträchtigen die Qualität des Biogases im Hinblick auf die verschiedenen Nutzungsmöglichkeiten von Biogas als Brennstoff in z. B. Verbrennungsmotoren oder aber auch als Brennstoff, der z. B. in das öffentliche Erdgasnetz mit einem möglichst hohen Methangasgehalt eingespeist werden soll. So ist z. B. Schwefelwasserstoff toxisch und sehr korrosiv und greift daher Komponenten und Bauteile von Blockhelzkraftwerken (BHKW's) an, wodurch sich deren Lebensdauer verringert. Ammoniak ist ebenfalls ein korrosives Gas, das zudem zu einem Stickstoffeintrag führt, das in den Verbrennungsabgasen der BHKW's zur Bildung von Stickoxiden (NOX) führt, die aus den bekannten Gründen unerwünscht sind.

Es ist daher bereits allgemein bekannt, das aus den Fermentern von Biogasanlagen erhaltene Biogas vor dessen energetischer Verwertung zu reinigen. So ist beispielsweise aus der gattungsbildenden DE 101 19 991 A1 ein Verfahren und eine Vorrichtung zur Reinigung von Biogas bekannt, bei dem aus dem ungereinigten Biogas mittels entsprechender Absorptionskolonnen Schwefelwasserstoff und Ammoniak in nachgeschalteten Reinigungsstufen zumindest teilweise entfernt werden.

Konkret wird hier in zwei hintereinandergeschalteten Reinigungsstufen, in denen jeweils zwei Schwefelwasserstoff-Absorptionskolonnen und zwei Ammoniak-Absorptionskolonnen vorgesehen sind, zuerst der Schwefelwasserstoff (H₂S) und anschließend in der zweiten Reinigungsstufe Ammoniak (NH₃) ausgewaschen. Zum Auswaschen von Schwefelwasserstoff wird dem ungereinigten Biogas Natronlauge (NAOH-Lösung) als alkalische Waschlösung zugeführt, wodurch der Schwefelwasserstoff zu Natriumsulfit (Na₂S) und Wasser reagiert. Dabei soll durch die Zugabe von Natronlauge ferner auch das im Biogas enthaltene Kohlendioxid (CO₂) zu Natriumcarbonat (Na₂CO₃) und Wasser reagieren. Da das freie Absorbens Natronlauge bei einer derartigen Verfahrensführung regelmäßig sofort vom Kohlendioxid umgesetzt wird, muss hier sehr viel Natronlauge zugeführt werden, damit ausreichend Natriumcarbonat gebildet wird, das mit dem noch verbliebenen Schwefelwasserstoff chemisch zu Natriumsulfit (Na₂S), Kohlendioxid und Wasser reagiert. Dies erfordert nachteiliger Weise einen hohen Verbrauch an Natronlauge und bewirkt zudem insbesondere im Hinblick auf schwankende Schwefelwasserstoffkonzentrationen im Biogas, dass der Schwefelwasserstoff gegebenenfalls nicht in dem erwünschten Maße ausgewaschen wird.

In der zweiten Reinigungsstufe wird dann den Ammoniak-Absorptionskolonnen als Lösungs- bzw. Waschmittel Schwefelsäure (H₂SO₄) zugeführt, das mit dem Ammoniak chemisch zu Ammoniumsulfat ((NH₄)₂SO₄) reagiert. Dieses Ammoniumsulfat kann in der Landwirtschaft als Dünger verwendet werden.

Es ist daher Aufgabe der vorliegenden Erfindung, ein alternatives Verfahren und eine alternative Gasreinigungsanlage zur Verfügung zu stellen, mittels dem bzw. mittels der eine Reinigung von Biogas funktions- und prozesssicher auf einfache Weise mit einem hohen Wirkungsgrad möglich ist.

Diese Aufgabe wird bezüglich des Verfahrens gelöst mit den Merkmalen des Patentanspruchs 1 und bezüglich der Gasreinigungsanlage gelöst mit den Merkmalen des Patentanspruchs 8. Weitere vorteilhafte Ausgestaltungen sind Gegenstand der darauf rückgezogenen Unteransprüche, deren Wortlaut hiermit ausdrücklich in die Beschreibung mit einbezogen wird.

Erfindungsgemäß ist vorgesehen, dem ungereinigten Biogas in einer Schwefelwasserstoff-Reinigungsstufe, die vorzugsweise durch wenigstens eine Schwefelwasserstoff-Absorptionskolonne gebildet ist, Wasserstoffperoxid (H₂O₂) zuzuführen, wodurch entsprechend der nachfolgenden Gleichung:

H₂S + 4 H₂O₂ → H₂SO₄ + 4 H₂O

durch die Reaktion von Wasserstoffperoxid (H₂O₂) und Schwefelwasserstoff (H₂S) neben Wasser auch Schwefelsäure (H₂SO₄) und Wasser (H₂O) gebildet wird, welches dann einer Ammoniak-Reinigungsstufe, die vorzugsweise durch wenigstens eine Ammoniak-Absorptionskolonne gebildet ist, zugeführt wird, in der dann entsprechend der nachfolgenden Gleichung:

2 NH₃ + H₂SO₄ → ((NH₄)₂SO₄)

durch die Reaktion von Ammoniak (NH₃) mit Schwefelsäure (H₂SO₄) Ammoniumsulfat ((NH₄) ₂SO₄) gebildet wird. Diese in der Ammoniak-Reinigungsstufe gebildeten Mengen an Ammoniumsulfat können dann in der Landwirtschaft als Dünger verwendet werden, wozu das Ammoniumsulfat beispielsweise in einem entsprechenden Speicherbehälter zwischengespeichert wird. Alternativ oder zusätzlich dazu kann das Ammoniumsulfat aber auch in das Endlager der Biogasanlage zurückgeführt werden, um nicht den Stickstoffgehalt in den anderen Fermentern in unerwünschter Weise zu erhöhen.

Die Gasreinigungsanlage weist für eine derartige Verfahrensführung eine Speicher- und Dosiereinrichtung für Wasserstoffperoxid auf, mittels der der Schwefelwasserstoff-Absorptionskolonne eine vorgegebene Menge an Wasserstoffperoxid zugeführt werden kann. Das Wasserstoffperoxid kann in Reinform oder aber auch in wässriger Lösung vorliegen. Zudem weist die Gasreinigungsanlage eine Speicher- und Dosiereinrichtung für von der Schwefelwasserstoff-Absorptionskolonne abgezogene Schwefelsäure auf, mittels der dann der Ammoniak-Absorptionskolonne eine vorgegebene Menge an Schwefelsäure zugeführt werden kann.

Mit einer derartigen erfindungsgemäßen Verfahrensführung und einer derartigen erfindungsgemäßen Gasreinigungsanlage kann somit die Reinigung des Biogases von den Bestandteilen Ammoniak und Schwefelwasserstoff auf einfache Weise lediglich durch die Bereitstellung von Wasserstoffperoxid als Lösungsmittel erreicht werden, so dass der Prozess insgesamt äußerst wirtschaftlich betrieben werden kann. Das für die Ammoniakauswaschung erforderliche Lösungsmittel Schwefelsäure wird dann prozessimmanent durch die Reaktion von Wasserstoffperoxid und Schwefelwasserstoff zu Schwefelsäure zur Verfügung gestellt. Des weiteren ist eine derartige Gasreinigungsanlage insgesamt mit relativ wenigen Bauteilen herstellbar sowie übersichtlich und einfach zu bedienen bzw. zu regeln, wobei aufgrund der zuvor geschilderten Prozessführung zudem auch eine hohe Prozesssicherheit im Hinblick auf eine nahezu vollständige Umsetzung und Entfernung von Ammoniak und Schwefelwasserstoff einfachst gewährleistet werden kann.

Gemäß einer besonders bevorzugten Weiterbildung der Erfindung ist vorgesehen, die Beseitigung des Schwefelwasserstoffes zweistufig durchzuführen, in dem der z. B. durch eine Schwefelwasserstoff-Absorptionskolonne gebildeten ersten Reinigungsstufe eine zweite Reinigungsstufe nachgeschaltet wird, die bevorzugt als Adsorptionsstufe, z. B. unter Verwendung von Aktivkohle, ausgeführt ist. Für die beiden Reinigungsstufen von Schwefelwasserstoff können dann unterschiedliche Schwefelwasserstoffkonzentrationsschwellwerte vorgegeben werden, wie dies mit Anspruch 3 beansprucht ist.

Als besonders vorteilhaft hat sich zudem herausgestellt, die Ammoniakentfernung in der Ammoniak-Reinigungsstufe bei einem pH-Wert der schwefelsauren Waschlösung von 2 bis 4 durchzuführen, da dadurch die Reinigung von Ammoniak immer stabil unter dem bevorzugten Schwellwert von 1 mg/Nm³ gehalten werden kann.

Das ungereinigte Biogas, das vorzugsweise, wie zuvor geschildert, von Schwefelwasserstoff und Ammoniak gereinigt ist, z. B. auf die zuvor beschriebene Art und Weise, kann weiter in einer Kohlendioxid-Reinigungsstufe von Kohlendioxid (CO₂) befreit werden. Bevorzugt ist hier aber eine Kombination dieser Kohlendioxid-Reinigungsstufe als dritte Reinigungsstufe mit den beiden zuvor ausführlich beschriebenen beiden ersten Reinigungsstufen, da sich hierdurch eine insgesamt funktionssichere, effektive und wirtschaftliche Auswaschung von Schwefelwasserstoff, Ammoniak und Kohlendioxid erreichen lässt.

Diese Kohlendioxid-Reinigungsstufe wird bevorzugt von wenigstens einer Kohlendioxid-Absorptionskolonne gebildet, in der bevorzugt mittels chemischer Absorption mit einer wässrigen Aminlösung als Absorptionsmittel, d. h. mittels einer Aminwäsche, dem Biogas Kohlendioxid entzogen wird. Mit einer derartigen Kohlendioxidwäsche, die vorzugsweise als Amin-Druckgaswäsche ausgelegt ist, kann jetzt der Methangehalt im Biogas gesteigert werden. Dazu wird das Absorptionsmittel mittels z. B. eines oder mehrerer Wärmetauscher bevorzugt auf eine Temperatur von 15°C oder darunter, höchst bevorzugt auf eine Temperatur von 10°C oder darunter abgekühlt. Damit wird sichergestellt, dass nur minimale Verluste an Absorptionsmittel auftreten und der Wassertaupunkt gering ist. Das am Kopf der bevorzugt als Amin-Druckgaswäscher ausgebildeten Kohlendioxid-Absorptionskolonne austretende, gereinigte Biogas kann dann z. B. direkt in ein an die Gasreinigungsanlage angeschlossenes Blockheizkraftwerk eingeleitet werden. Dazu sind entsprechende Rohrleitungen mit Pumpen und dergleichen vorzusehen. Bei einer Einspeisung des von Kohlendioxid gereinigten Biogases ihn ein Erdgasnetz ist zudem eine Entfeuchtung sowie eine Odorierung des Biogases vorteilhaft.

Gemäß einer weiteren vorteilhaften Weiterbildung ist vorgesehen, das von der Kohlendioxid-Absorptionskolonne sumpfseitig abgezogene, mit Kohlendioxid beladene Absorptionsmittel anschließend über eine Pump- und/oder Verdichtereinrichtung zu führen, in der das Absorptionsmittel verdichtet wird, z. B. auf bevorzugt 10 bar verdichtet wird, bevor es in eine bevorzugt als Stripper ausgebildete Desorptionsstufe eingebracht wird, in der durch Entspannung das gebundene Kohlendioxid aus dem Absorptionsmittel entfernt wird.

Der Druck im Stripper wird hier so eingestellt, dass eine optimale Entfernung des Kohlendioxides möglich ist. Gleichzeitig kann über entsprechende Wärmetauscher, insbesondere Thermalöl-Wärmetauscher, die zudem mit dem Blockheizkraftwerk thermisch gekoppelt sind, eine vorteilhafte Vorerwärmung des dem Stripper zugeführten und mit Kohlendioxid beladenen Absorptionsmittels erzielt werden. Besonders bevorzugt ist hier zudem eine Verfahrensführung und ein Anlagenkonzept bei dem das die Kohlendioxid-Absorptionskolonne verlassende, mit Kohlendioxid beladene Absorptionsmittel durch das demgegenüber heiße, den Stripper verlassende und nicht mehr mit Kohlendioxid beladene Absorptionsmittel vorgewärmt wird. Diese eben beschriebenen Möglichkeiten der thermischen Einkopplung und Auskopplung von Wärme in die unterschiedlichen Prozessströme kann sowohl einzeln als auch in beliebigen Kombinationen untereinander vorgesehen werden und dementsprechend wird jede einzelne Möglichkeit für sich alleine gesehen als vorteilhafte Weiterbildung der Erfindung beansprucht. Besonders bevorzugt ist eine Verfahrensführung, bei der das mit Kohlendioxid beladene Absorptionsmittel zweistufig vorgewärmt wird, nämlich einmal durch den eben beschriebenen heißen und nicht mehr mit Kohlendioxid beladenen Absorptionsmittelstrom, der zum Kopf der Kohlendioxid-Absorptionskolonne geführt wird, und andererseits durch die ebenfalls zuvor beschriebene thermische Einkopplungsmöglichkeit von Abwärme des Blockheizkraftwerks mittels z. B. eines Thermalöl-Wärmetauschers.

Ein besonderer Vorteil einer derartigen Verfahrensführung besteht darin, dass hier keine mit der üblicherweise bei Biogas verwendeten Druckwechseladsorption vergleichbaren Methanverluste auftreten können.

Gemäß einer weiteren bevorzugten Weiterbildung ist vorgesehen, am Kopf des Strippers eine Kondensation des mitgerissenen Wassers und Waschmittels vorzusehen, so dass ein sehr reines Kohlendioxid erhalten wird, das gemäß einer weiteren besonders vorteilhaften Erfindungsvariante einer Verflüssigung in z. B. weiteren Wärmetauschern zugeführt werden kann, in denen mit Kaltwasser von z. B. -15°C die Verflüssigung vorgenommen wird. Die Bereitstellung der Kälteleistung wird z. B. durch einen luftgekühlten Kaltwassersatz vorgenommen. Das so kondensierte, flüssige Kohlendioxid kann dann einem Lagertank zugeführt werden oder aber auch in handelsübliche CO₂-Flaschen abgefüllt werden. Dieser so gewonnene CO₂ kann z. B. in Verbindung mit einem Peletierer zur Erzeugung von Trockeneis verwendet werden. Auch weitere Verwertungen, z. B. zur Verwendung in Gewächshäusern sind möglich.

Die Absorptionskolonnen sind bevorzugt Füllkörper-Kolonnen.

Gemäß einer weiteren bevorzugten Ausgestaltung der vorliegenden Erfindung ist vorgesehen, dass dem Wasserstoffperoxid in der Schwefelwasserstoff-Reinigungsstufe eine solche Menge an Natronlauge zugegeben, insbesondere zugeimpft wird, dass der pH-Wert in der Schwefelwasserstoff-Reinigungsstufe auf einen gewünschte, vorgegebenen Wert eingestellt werden kann. Insbesondere wird hier ein schwach basischer pH-Wert bevorzugt, der zwischen 7 und 10, höchst bevorzugt zwischen 7 und 8,5 liegen sollte. Damit lassen sich in der Schwefelwasserstoff-Reinigungsstufe hervorragende Ergebnisse bezüglich der Schwefelwasserstoff-Auswaschung erzielen. Die Natronlauge wird hier bevorzugt über eine separate Speicher- und Dosiereinrichtung zugedüst, und zwar bevorzugt außerhalb der Schwefelwasserstoff-Absorptionskolonne, d. h. entweder im Bereich einer zwischen dem Sumpf und dem Kopf der Schwefelwasserstoff-Absorptionskolonne verlaufenden Rohrleitung oder im Bereich einer Zuführleitung von H₂O₂ zu eben dieser Sumpf- und Kopf-Rohrleitung. Das Eindüsen kann hier beispielsweise über eine geeignete Einmisch- bzw. Einspritzventilanordnung erfolgen.

Gemäß einer weiteren besonders bevorzugten Ausgestaltung der vorliegenden Erfindung ist vorgesehen, dass der Kohlendioxid-Reinigungsstufe eine Adsorptionsstufe nachgeschaltet ist. Diese Kohlendioxid-Reinigungsstufe ist bevorzugt durch einen Adsorber gebildet, der als Adsorptionsmittel Aktivkohle aufweist. Mit dieser Adsorptionsstufe können Restspuren von Kohlendioxid, von Schwefelverbindungen und von organischen Stickstoffverbindungen nochmals weiter verringert bzw. beseitigt werden, so dass ein Methanstrom mit einem sehr hohen Reinheitsgrad erhalten wird. Diese der Kohlendioxid-Reinigungsstufe nachgeschaltete Adsorptionsstufe kann sowohl zusätzlich als auch alternativ zur zuvor beschriebenen Adsorptionsstufe nach der Schwefelwasserstoff-Reinigungsstufe vorgesehen sein. Falls die Adsorptionsstufe nach der Schwefelwasserstoff-Reinigungsstufe nicht vorgesehen sein sollte, wäre gegebenenfalls die Adsorptionsstufe nach der Kohlendioxid-Reinigungsstufe entsprechend größer auszulegen.

Gemäß einem weiteren erfindungsgemäßen Aspekt ist der Kohlendioxid-Reinigungsstufe, insbesondere einer der Kohlendioxid-Reinigungsstufe nachgeschalteten Desorptionsstufe, eine weitere, zweite Kohlendioxid-Reinigungsstufe nachgeschaltet, in der dem mittels der ersten Kohlendioxid-Reinigungsstufe vorgereinigten Kohlendioxidstrom Wasserstoffperoxid als Reinigungsmittel zugeführt wird. Dieses Wasserstoffperoxid wird bevorzugt mittels der der Schwefelwasserstoff-Absorptionskolonne zugeordneten Speicher- und Dosiereinrichtung zur Verfügung gestellt. Mittels dieser Wasserstoffperoxid-Wäsche können dem Kohlendioxidstrom eventuell noch vorhandene Spuren von Schwefelwasserstoff entzogen werden, was zu einem besonders reinen Kohlendioxidstrom führt. Dieses Kohlendioxid hat dann eine sogenannte Lebensmittelqualität und kann z. B. zur Trockeneisherstellung Verwendung finden.

Die Erfindung wird nachfolgend anhand einer Zeichnung näher erläutert.

Es zeigen:
- Fig. 1: schematisch ein Blockschaltbild einer mit einer Biogasanlage gekoppelten Gasreinigungsanlage,
- Fig. 2: schematisch den Aufbau und die Verfahrensführung der Gasreinigungsanlage der Fig. 1 im Detail,
- Fig. 3: schematisch eine Gasreinigungsanlage entsprechend der Fig. 2 mit zusätzlichem Aktivkohlefilter und einer Zudosierungsmöglichkeit von Natronlauge, und
- Fig. 4: schematisch eine Gasreinigungsanlage mit zusätzlicher Kohlendioxid-Wäsche.

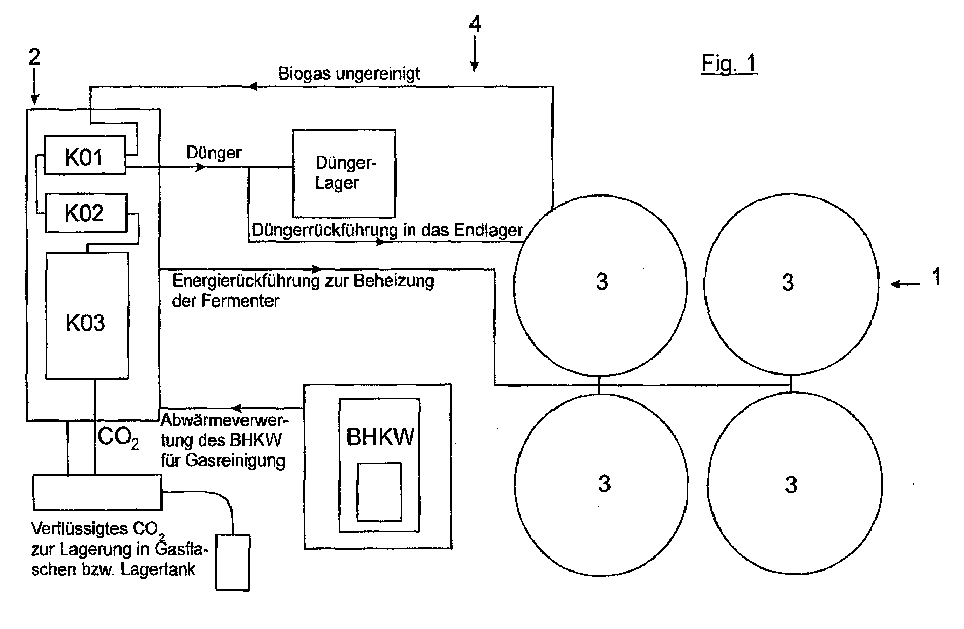

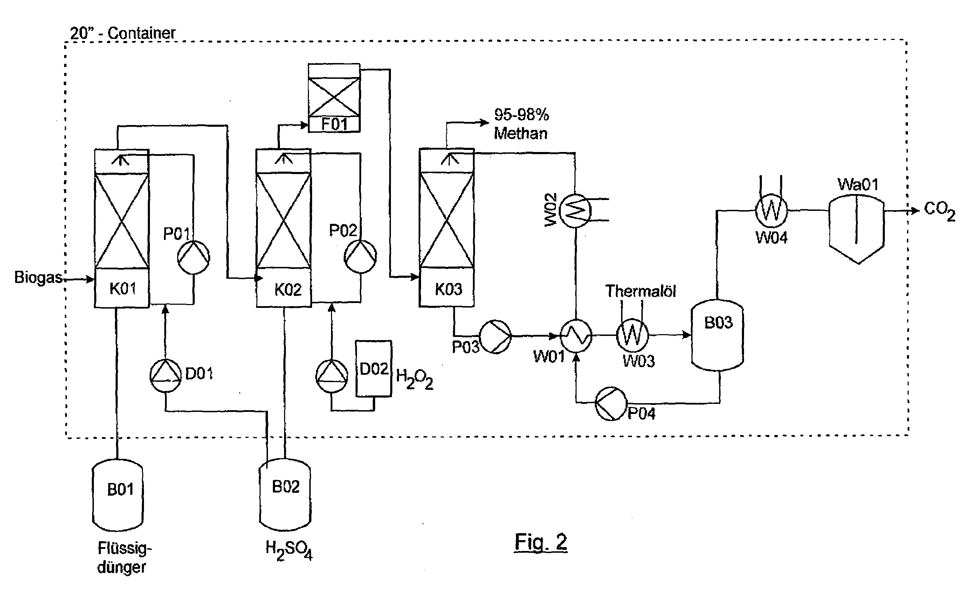

In Fig. 1 ist schematisch eine mit einer Biogasanlage 1 gekoppelte Gasreinigungsanlage 2 gezeigt. Die Gasreinigungsanlage 2 ist schematisch mit mehr Details in der Fig. 2 dargestellt, die hier beispielhaft in einem Container, z. B. einem 20"-Container installiert ist, wie dies in der Fig. 2 schematisch durch die strichlierte Linie angedeutet ist.

Die Biogasanlage 1 selbst weist wiederum mehrerer Fermenterbehälter 3 auf, die die eigentlichen Fermenter, Nachfermenter oder Endlager bilden und die an sich bekannter Bauart sind, so dass darauf nicht mehr näher eingegangen wird. Das in der Biogasanlage 1 gewonnene, ungereinigte Biogas gelangt über eine Rohrleitung 4 in die Gasreinigungsanlage 2 und wird dort im Sumpf einer Ammoniak-Absorptionskolonne K01 zugeführt und durchläuft dort in Richtung zum Kopf der Ammoniak-Absorptionskolonne K01 eine Füllkörperschüttung, und zwar im Gegenstrom zu einer im Kopf der Ammoniak-Absorptionskolonne aufgegebenen Schwefelsäure (H₂SO₄) als Waschlösung, die mittels einer Pumpe P01 im Kreislauf zwischen Sumpf und Kopf der Ammoniak-Absorptionskolonne gepumpt wird. Das Biogas wird vorzugsweise auf 50 mbar vorverdichtet zugeführt. Nach der Biogasanlage steht es regelmäßig mit einem Druck von ca. 10 mbar zur Verfügung. Die Verdichtung erfolgt mittels eines geeigneten Verdichters. Diese Pumpe P01 bildet Bestandteil einer Speicher- und Dosiereinrichtung, die einen Speicherbehälter B02 für Schwefelsäure aufweist, aus der in Abhängigkeit von vorgegebenen Zudosierparametern zu gegebenen Zeitpunkten kontinuierlich bzw. auch diskontinuierlich eine vorgegebene Menge an Schwefelsäure mittels der Dosierpumpe D01 abgezogen und in den Absorptionsmittelkreislauf der Ammoniak-Absorptionskolonne K01 zudosiert wird, insbesondere um evt. Verluste von Schwefelsäure auszugleichen.

In der Ammoniak-Absorptionskolonne K01 reagiert die Schwefelsäure mit dem Ammoniak zu Ammoniumsulfat, das in einem Speicherbehälter B01 als Flüssigdünger für die Landwirtschaft zwischengespeichert wird. Das Ammoniumsulfat wird durch entsprechende Mittel in bzw. außerhalb der Ammoniak-Absorptionskolonne K01 vom Absorptionsmittel (H₂SO₄) getrennt und wie zuvor beschrieben in den Speicherbehälter B01 abgeführt. Alternativ oder zusätzlich dazu kann das Ammoniumsulfat aber auch in das Endlager der Biogasanlage 1 zurückgeführt werden, wie dies schematisch in Fig. 1 gezeigt ist.

Die Ammoniakentfernung erfolgt hierbei bei einem pH-Wert von 2 bis 4 der Waschlösung Schwefelsäure, um sicherzustellen, dass das aus der Wasch- bzw. Ammoniak-Absorptionskolonne K01 austretende Biogas von Ammoniak gereinigt ist und die Reingaskonzentration immer stabil unter dem Wert von bevorzugt 1 mg/Nm³ liegt, egal wie hoch die Eintrittskonzentration von Ammoniak ist.

Der Waschkreislauf wird dabei mit einer pH-Messung überwacht, die hier nicht im Detail dargestellt ist.

Das so von Ammoniak gereinigte Biogas wird dann anschließend dem Sumpf einer der Ammoniak-Absorptionskolonne K01 nachgeschalteten Schwefelwasserstoff-Absorptionskolonne K02 zugeführt, wo es in Richtung zum Kopf der Schwefelwasserstoff-Absorptionskolonne K02 eine Füllkörperschüttung durchströmt, auf die Wasserstoffperoxid (H₂O₂) als Waschlösung bzw. Absorptionsmittel aufgegeben wird, das die Füllkörperschüttung im Gegenstrom zum von Ammoniak gereinigten Biogas durchströmt und aus dem so vorgereinigten Biogas Schwefelwasserstoff (H₂S) entzieht, da Wasserstoffperoxid (H₂O₂) und Schwefelwasserstoff (H₂S) zu Schwefelsäure (H₂SO₄) reagieren, das im Sumpf der Schwefelwasserstoff-Absorptionskolonne K02 abgezogen und im Speicherbehälter B02 als Waschlösung bzw. Absorptionsmittel für die Ammoniak-Absorptionskolonne K01 zwischengespeichert wird.

Die Zudosierung von Wasserstoffperoxid zur Schwefelwasserstoff-Absorptionskolonne K02 erfolgt mittels einer Speicher- und Dosiereinrichtung, die einen Speicherbehälter D02 für das Wasserstoffperoxid und eine damit gekoppelte Dosierpumpe aufweist, mittels der eine Zudosierung in den Absorptionsmittelkreislauf der Schwefelwasserstoff-Absorptionskolonne K02 entsprechend vorgegebener Zudosierparameter zuvor gegebenen Zeiten zu vorgegebenen Mengen erfolgt. In diesem Kreislauf ist analog zu Ammoniak-Absorptionskolonne eine Kreislaufpumpe P02 vorgesehen.

In der Schwefelwasserstoff-Absorptionskolonne K02 wird dem Biogas Schwefelwasserstoff bis zu einer hier beispielhaft vorgegebenen Grenzkonzentration von etwa 50 mg/Nm³ entzogen. Dieses so teilweise vom Schwefelwasserstoff befreite und vorgereinigte Biogas gelangt dann über den Kopf der Schwefelwasserstoff-Absorptionskolonne K02 in einen als Aktivkohlefilter ausgebildeten Adsorber F01, in dem der sich noch im Biogas befindliche Schwefelwasserstoff (H₂S) auf Reingaskonzentrationen nachgereinigt wird, die bevorzugt bei unter 3 mg/Nm³ liegt.

Auch der Waschkreislauf der Schwefelwasserstoff-Absorptionskolonne K02 wird mittels einer pH-Messung und einer Redoxmessung überwacht. Besonders vorteilhaft liegt hier der pH-Wert für zufriedenstellende Ergebnisse im sauren Bereich, z. B. zwischen 2 und 5. Dies hängt im einzelnen von der Biogaszusammensetzung ab.

Das so von Ammoniak und Schwefelwasserstoff vorgereinigte Biogas gelangt dann von dem Aktivkohlefilter F01 in den Sumpf eines Amin-Druckgaswäschers K03, wo es im Gegenstrom zu einer wässrigen Aminlösung als Waschlösung bzw. Absorptionsmittel wiederum eine Füllkörperschüttung in Richtung zum Kopf des Druckgaswäschers K03 durchströmt.

Dadurch wird das Biogas von Kohlendioxid befreit, so dass das mit Kohlendioxid beladene Absorptionsmittel über den Sumpf des Druckgaswäschers K03 mittels einer Pumpe P03 auf z. B. 10 bar verdichtet wird und anschließend einem Stripper B03 zugeleitet wird. Bevor das mit Kohlendioxid beladene Absorptionsmittels allerdings in den Stripper B03 gelangt, wird es in einer ersten Wärmetauscherstufe W01, in der ein heißer vom Stripper B03 kommender und vom Kohlendioxid befreiter Absorptionsmittelstrom Wärme abgibt, und einer zweiten Wärmetauscherstufe W03, die durch einen Thermalöl-Wärmetauscher gebildet ist, aufgeheizt und zwar beispielsweise bevorzugt auf Temperaturen von 165 bis 175°C. In dem Stripper B03 wird dann durch Entspannung das gebundene Kohlendioxid aus dem Absorptionsmittel entfernt. Im als Kondensator ausgebildeten Wärmetauscher W04 erfolgt am Kopf des Strippers B03 eine Kondensation des mitgerissenen Wassers und Absorptionsmittels, wobei die am Kondensator W04 anfallende Kondensationswärme dann zur Beheizung der z. B. Fermenter zurück zur Biogasanlage 1 geführt wird bzw. anderweitig in die Biogasanlage thermisch eingekoppelt wird.

Der Druck im Stripper B01 wird dabei so eingestellt, dass eine optimale Entfernung des Kohlendioxids erfolgt.

Bei einer Erwärmung auf Temperaturen von bevorzugt 165 bis 175°C wird sichergestellt, dass überwiegend Kohlendioxid und wenig Wasser verdampft.

Mittels der Pumpe P04 wird dann die im Stripper B03 entspannte und von Kohlendioxid befreite Waschlösung als gereinigtes Absorptionsmittel über die Wärmetauscher W01 und W02 wiederum zum Druckgaswäscher K03 geleitet. Über den Betriebsdruck des Strippers und aufgrund der Abkühlung im Wärmetauscher W02 kann die gewünschte Kohlendioxidbeladung im Biogas von unter 0,05 Vol.% bis maximal 6 Vol.% eingestellt werden.

Zur Erhitzung des Thermalöls des Wärmetauschers W03 wird, wie dies in der Fig. 1 schematisch dargestellt ist, die Abwärme des Blockheizkraftwerks (BHKW) oder anderer Wärmequellen wie z. B. Pellets oder Holzhackschnitzel verwendet. Die Abwärme der entsprechenden Heizquelle wird über Abgas-Wärmetauscher und entsprechende Wärmeleitungen an den Thermalölkessel abgegeben. Diese Wärme wird im Stripper B03 für die Erhitzung des CO₂-Aminkomplexes auf ca. 165 bis 195°C verwendet. Dadurch wird die Zerlegung des CO₂-Aminkomplexes in die Bestandteile CO₂ und wässrige Aminlösung möglich. Aus beiden Volumenströmen wird über weitere Wärmetauscher W02, W04 die Wärme zurückgewonnen und über ein Heizleitungssystem der Biogasanlage, insbesondere einem oder mehreren der Fermenter zugeführt.

Das aus dem Kondensator W04 austretende Kohlendioxid kann dann der Verflüssigung in einer entsprechenden Verflüssigungsvorrichtung WA01 zugeführt werden, in dem z. B. mittels Kaltwasser von z. B. -15°C die Verflüssigung erfolgt. Die Bereitstellung der Kälteleistung kann durch einen luftgekühlten Kaltwassersatz vorgenommen werden. Das kondensierte, flüssige Kohlendioxid kann dann wiederum einem Lagertank zugeführt werden, von dem aus z. B. ein Peletierer zur Erzeugung von Trockeneis bedient werden kann.

Durch diese Kohlendioxid-Reduzierung in einem regenerativen Prozess besteht somit gemäß einer besonders vorteilhaften Auswirkung der Erfindung die Möglichkeit, dieses Kohlendioxid einem weiteren Prozess zur Verfügung zu stellen, z. B. der Trockeneisherstellung, so dass dadurch ein Handel mit Klimazertifikaten möglich wird.

Wie dies der Fig. 1 und der Fig. 2 zudem weiter entnommen werden kann, kann das bevorzugt auf einen Methangehalt von 95 bis 98 % gereinigte Biogas dann einem Blockheizkraftwerk (BHKW) zugeführt werden, wo es in z. B. Verbrennungsmotoren zur Stromerzeugung verbrannt wird. Alternativ oder zusätzlich dazu kann das Biogas aber auch als sog. Biomethan einem Erdgasnetz zugeführt werden. Vor der Einspeisung in das öffentliche Erdgasnetz hat hier jedoch eine Aufstreckung des Biomethans auf den vom Betreiber geforderten Reinheitsgrad und/oder Heizwert stattzufinden, wobei das Biomethan vorher vorzugsweise entfeuchtet und odoriert wird, um den typischen Erdgasgeruch zu erzeugen. Dabei wird das auf Erdgasqualität aufgereinigte Biogas durch entsprechende Messgeräte geprüft und je nach Anforderung des zu beschickenden Gasnetzes auf z. B. 50 bar verdichtet. Die Einspeisung selbst erfolgt über einen für die Gasverdichtung geeigneten Kompressor.

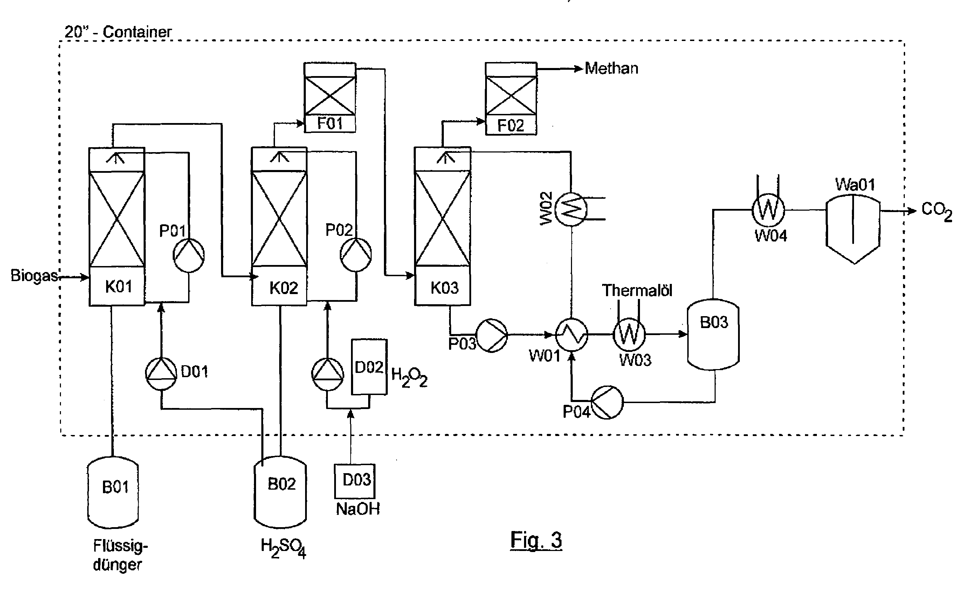

In der Fig. 3 ist eine im Wesentlichen der Darstellung der Fig. 2 entsprechende Verfahrensführung der erfindungsgemäßen Gasreinigungsanlage gezeigt. Im Unterschied zur Verfahrensführung der Fig. 2 ist jedoch hier dem Druckgaswäscher K03 ein als Aktivkohlefilter ausgebildeter Adsorber F02 nachgeschaltet, mittels dem noch im Biogas befindliche Restspuren von Kohlendioxid, von Schwefelverbindungen und von organischen Stickstoffverbindungen beseitigt werden können.

Im weiteren Unterschied zur Verfahrensführung gemäß der Fig. 2 ist hier zudem ein Speicherbehälter D03 für Natronlauge (NaOH) vorgesehen, mittels dem dem Wasserstoffperoxid (H₂O₂) der Schwefelwasserstoff-Absorptionskolonne K02 eine solche Menge an Natronlauge zudosiert werden kann, dass sich in der Schwefelwasserstoff-Absorptionskolonne K02 ein bevorzugter pH-Wert zwischen 7 bis 8,5 einstellt. Wie dies in der Fig. 3 lediglich äußerst schematisch dargestellt ist, kann die Zudosierung bzw. die Zudüsung der Natronlauge hier im Bereich einer H₂O₂-Zuführleitung erfolgen, mittels der H₂O₂ bzw. ein Gemisch aus H₂O₂ und NaOH einer zwischen Kopf und Sumpf der Schwefelwasserstoff-Absorptionskolonne verlaufenden Rohrleitung zugeführt werden kann. Alternativ dazu kann die Eindüsung aber auch in die zwischen Sumpf und Kopf der Schwefelwasserstoff-Absorptionskolonne verlaufende Rohrleitung erfolgen.

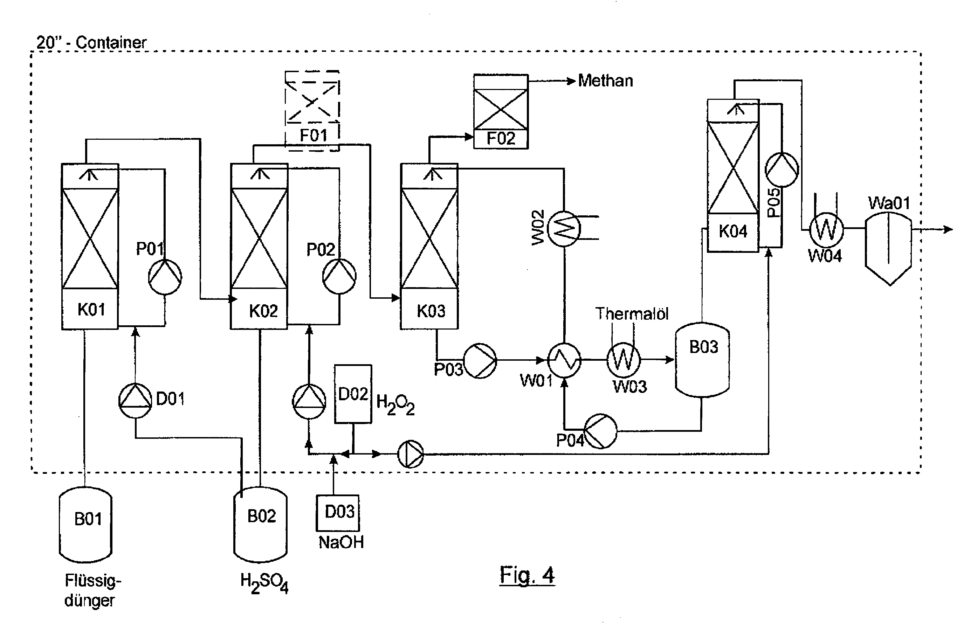

In der Fig. 4 ist schließlich eine weitere alternative Ausführungsform dargestellt, die im Wesentlichen der Verfahrensführung gemäß Fig. 3 entspricht. Im Unterschied zur Verfahrensführung gemäß Fig. 3 ist jedoch hier der Aktivkohlefilter F01 lediglich strichliert dargestellt, was bedeutet, dass dieser Aktivkohlefilter F01 gegebenenfalls auch nicht vorhanden sein kann, falls der Aktivkohlefilter F02 mit einer entsprechenden Dimensionierung vorgesehen ist. Diese Option kann auch in Verbindung mit den Ausführungen der Fig. 2 und 3 vorgesehen sein. Bevorzugt ist jedoch das Vorsehen von sowohl dem Aktivkohlefilter F01 als auch dem Aktivkohlefilter F02.

Um insbesondere das Kohlendioxid nach dem Stripper B03 auf einen noch höheren Reinheitsgrad zu reinigen, so dass auf einfache und funktionssichere Weise eine Lebensmittelqualität für das CO₂ erzielt werden kann, ist in der Fig. 4 gemäß einer alternativen Verfahrensführung vorgesehen, dem Stripper B03 eine Kohlendioxid-Absorptionskolonne K04 nachzuschalten, der im Sumpf das aus dem Stripper B03 gewonnene Kohlendioxid zugeführt wird, das dann in der Kohlendioxid-Absorptionskolonne K04 in Richtung zum Kopf der Kohlendioxid-Absorptionskolonne K04 eine Füllkörperschüttung durchläuft und zwar im Gegenstrom zum im Kopf der Kohlendioxid-Absorptionskolonne K04 aufgegebenen Wasserstoffperoxid (H₂O₂), welches dem Kohlendioxidstrom insbesondere Restspuren von Schwefelwasserstoff entzieht. Dadurch kann am Kopf der Kohlendioxid-Absorptionskolonne K04 ein hochreiner Kohlendioxidstrom abgezogen werden, der beispielsweise, wie dies bereits zuvor in Verbindung mit der Fig. 2 näher erläutert worden ist, über einen Kondensator W04 einer Verflüssigungsvorrichtung WA01 zugeführt werden kann.

Das Reinigungsmittel H₂O₂ wird hier mittels einer Pumpe P05 im Kreislauf zwischen Sumpf und Kopf der Kohlendioxid-Absorptionskolonne K04 gepumpt, wobei die Zudosierung von Wasserstoffperoxid zur Kohlendioxid-Absorptionskolonne K04 hier ebenfalls mittels der der Schwefelwasserstoff-Absorptionskolonne K02 zugeordneten Speicher- und Dosiereinrichtung erfolgt, die den Speicherbehälter D02 für das Wasserstoffperoxid und eine damit gekoppelte Dosierpumpe aufweist.

## Patentansprüche

1. Verfahren zur Reinigung von Biogas einer Biogasanlage, bei dem aus dem ungereinigten Biogas Schwefelwasserstoff (H₂S) und Ammoniak (NH₃) zumindest teilweise entfernt werden,
**dadurch gekennzeichnet,**
**dass** dem Biogas in einer Schwefelwasserstoff-Reinigungsstufe (K02) Wasserstoffperoxid (H₂O₂) zugeführt wird, und
**dass** die durch die Reaktion von Waserstoffperoxid (H₂O₂) und Schwefelwasserstoff (H₂S) in der Schwefelwasserstoff-Reinigungsstufe (K02) gebildete Schwefelsäure (H₂SO₄) wenigstens teilweise einer Ammoniak-Reinigungsstufe (K01) zugeführt wird, in der durch die Reaktion von Ammoniak (NH₃) mit Schwefelsäure (H₂SO₄) Ammoniumsulfat gebildet wird.

2. Verfahren nach Anspruch 1, **dadurch gekennzeichnet, dass** der Schwefelwasserstoff-Reinigungsstufe (K02) eine Adsorptionsstufe (FO1) als Sicherheitsfilter nachgeschaltet ist, so dass das Biogas zweistufig auf oder unter einen vorgegebenen Schwefelwasserstoffgrenzwert gereinigt wird.

3. Verfahren nach Anspruch 2, **dadurch gekennzeichnet,**
**dass** das Biogas in der Schwefelwasserstoff-Reinigungsstufe (K01) auf eine Schwefelwasserstoffkonzentration von 40 bis 60 mg/Nm³, bevorzugt von 50 mg/Nm³ vorgereinigt wird, und
**dass** das so vorgereinigte Biogas anschließend in der Adsorptionsstufe (F01) auf eine Schwefelwasserstoffkonzentration von kleiner oder gleich 5 mg/Nm³, bevorzugt von kleiner oder gleich 3 mg/Nm³ nachgereinigt wird.

4. Verfahren nach einem der Ansprüche 1 bis 3, **dadurch gekennzeichnet, dass** die Ammoniakentfernung in der Ammoniak-Reinigungsstufe (K01) bei einem pH-Wert der Schwefelsäure (H₂SO₄) aufweisenden Waschlösung von 2 bis 4 durchgeführt wird.

5. Verfahren nach einem der Ansprüche 1 bis 4, **dadurch gekennzeichnet, dass** die Zudosiermenge der Schwefelsäure (H₂SO₄) so vorgegeben wird, dass der Ammoniakanteil im Biogas nach der Ammoniak-Reinigungsstufe (K01) kleiner oder gleich 1 mg/Nm³ ist.

6. Verfahren nach einem der Ansprüche 1 bis 5, **dadurch gekennzeichnet, dass** die Schwefelwasserstoff-Reinigungsstufe (K02) der Ammoniak-Reinigungsstufe (K01) nachgeschaltet ist.

7. Verfahren nach einem der Ansprüche 1 bis 6, **dadurch gekennzeichnet, dass** dem Wasserstoffperoxid (H₂O₂) eine solche Menge an Natronlauge (NaOH) zugegeben bzw. zugeimpft wird, dass der pH-Wert in der Schwefelwasserstoff-Reinigungsstufe (K02) einen vorgegebenen Wert, insbesondere einen pH-Wert zwischen 7 und 10, höchst bevorzugt zwischen 7 und 8,5, aufweist.

8. Gasreinigungsanlage zur Reinigung von Biogas einer Biogasanlage, insbesondere nach einem Verfahren gemäß einem der Ansprüche 1 bis 7,
mit einer Schwefelwasserstoff-Absorptionskolonne zur wenigstens teilweisen Entfernung von Schwefelwasserstoff (H₂S) aus einem ungereinigten Biogas und mit einer Ammoniak-Absorptionskolonne zur wenigstens teilweisen Entfernung von Ammoniak (NH₃) aus dem ungereinigten Biogas,
**dadurch gekennzeichnet,**
**dass** die Gasreinigungsanlage eine Speicher- und Dosiereinrichtung (D02) für Wasserstoffperoxid (H₂O₂) aufweist, mittels der der Schwefelwasserstoff-Absorptionskolonne (K02) eine vorgegebene Menge Wasserstoffperoxid (H₂O₂) zuführbar ist, und
**dass** die Gasreinigungsanlage (K01) eine Speicher- und Dosiereinrichtung (B02, D01) für von der Schwefelwasserstoff-Absorptionskolonne (K01) abgezogene Schwefelsäure (H₂SO₄) aufweist, mittels der der Ammoniak-Absorptionskolonne (K01) eine vorgegebene Menge Schwefelsäure (H₂SO₄) zuführbar ist.

9. Gasreinigungsanlage nach Anspruch 8, **dadurch gekennzeichnet, dass** die Speicher- und Dosiereinrichtungen jeweils wenigstens einen Speicherbehälter (D02, B02) und wenigstens eine Dosierpumpe (D01) aufweisen.

10. Gasreinigungsanlage nach Anspruch 8 oder Anspruch 9, **dadurch gekennzeichnet, dass** jede Absorptionskolonne (K01, K02) eine Kreislaufpumpe (P01, P02) zwischen Sumpf und Kopf aufweist, mittels der die jeweilige Waschlösung vom Sumpf in den Kopf pumpbar ist, wo die Waschlösung auf eine Füllkörperschüttung aufgebbar ist, die im Gegenstrom vom ungereinigten Biogasstrom durchströmbar ist.

11. Gasreinigungsanlage nach einem der Ansprüche 8 bis 10, **dadurch gekennzeichnet, dass** der Schwefelwasserstoff-Absorptionskolonne (K02) ein Adsorber (F01) nachgeschaltet ist.

12. Gasreinigungsanlage nach Anspruch 11, **dadurch gekennzeichnet, dass** der Adsorber (F01) Aktivkohle als Adsorptionsmittel aufweist.

13. Gasreinigungsanlage nach einem der Ansprüche 8 bis 12, **dadurch gekennzeichnet,**
**dass** die Schwefelwasserstoff-Absorptionskolonne (K02) der Ammoniak-Absorptionskolonne (K01) nachgeschaltet ist, und
**dass** der Ammoniak-Absorptionskolonne (K01) ein Speicherbehälter (B01) für Ammoniumsulfat ((NH₄)₂ SO₄) nachgeschaltet ist.

14. Gasreinigungsanlage nach einem der Ansprüche 8 bis 13, **dadurch gekennzeichnet, dass** die Gasreinigungsanlage eine Speicher- und Dosiereinrichtung (D03) für Natronlauge (NaOH) aufweist, mittels der dem der Schwefelwasserstoff-Absorptionskolonne (K02) zuzuführenden Wasserstoffperoxid (H₂O₂) in Abhängigkeit vom einzustellenden pH-Wert in der Schwefelwasserstoff-Absorptionskolonne (K02) eine vorgegebene Menge an Natronlauge (NaOH) zuführbar ist.

15. Gasreinigungsanlage nach Anspruch 14, **dadurch gekennzeichnet, dass** die vorgegebene Menge Natronlauge (NaOH) im Bereich einer Sumpf- und Kopf-Rohrleitung zwischen Sumpf und Kopf der Schwefelwasserstoff-Absorptionskolonne (K02) oder im Bereich einer H₂O₂-Zuführleitung zur Sumpf- und Kopf-Rohrleitung zuführbar, insbesondere mittels einer Düseneinrichtung zudüsbar ist.

## Claims

1. Method for purifying biogas from a biogas installation, in which hydrogen sulphide (H₂S) and ammonia (NH₃) are at least partly removed from the impure biogas,
**characterized**
**in that** hydrogen peroxide (H₂O₂) is added to the biogas in a hydrogen sulphide purification stage (K02), and
**in that** the sulphuric acid (H₂SO₄) formed by the reaction of hydrogen peroxide (H₂O₂) and hydrogen sulphide (H₂S) in the hydrogen sulphide purification stage (K02) is at least partly fed to an ammonia purification stage (K01) in which ammonium sulphate is formed by the reaction of ammonia (NH₃) with sulphuric acid (H₂SO₄).

2. Method according to Claim 1, **characterized in that** an adsorption stage (F01) is connected as safety filter downstream of the hydrogen sulphide purification stage (K02), so that the biogas is purified in two stages to or below a specified hydrogen sulphide limit.

3. Method according to Claim 2, **characterized in**
**that** the biogas in the hydrogen sulphide purification stage (K01) is prepurified to a hydrogen sulphide concentration of 40 to 60 mg/m³ (S.T.P), preferably of 50 mg/m³ (S.T.P), and
in that the biogas thus prepurified is then subsequently purified in the adsorption stage (F01) to a hydrogen sulphide concentration of less than or equal to 5 mg/m³ (S.T.P), preferably of less than or equal to 3 mg/m³ (S.T.P).

4. Method according to any of Claims 1 to 3, **characterized in that** the ammonia removal in the ammonia purification stage (K01) is carried out at a pH of 2 to 4 of the wash solution comprising sulphuric acid (H₂SO₄).

5. Method according to any of Claims 1 to 4, **characterized in that** the amount of sulphuric acid (H₂SO₄) metered in is specified so that the proportion of ammonia in the biogas after the ammonia purification stage (K01) is less than or equal to 1 mg/m³ (S.T.P.).

6. Method according to any of Claims 1 to 5, **characterized in that** the hydrogen sulphide purification stage (K02) is connected downstream of the ammonia purification stage (KO1).

7. Method according to any of Claims 1 to 6, **characterized in that** sodium hydroxide (NaOH) is added to the hydrogen peroxide (H₂O₂) or the hydrogen peroxide (H₂O₂) is seeded with sodium hydroxide (NaOH) in an amount such that the pH in the hydrogen sulphide purification stage (K02) has a specified value, in particular a pH between 7 and 10, most preferably between 7 and 8.5.

8. Gas purification system for purifying biogas of a biogas installation, in particular by a method according to any of Claims 1 to 7,
comprising a hydrogen sulphide absorption column for at least partial removal of hydrogen sulphide (H₂S) from an impure biogas and comprising an ammonia absorption column for at least partial removal of ammonia (NH₃) from the impure biogas,
**characterized**
**in that** the gas purification system has a storage and metering device (D02) for hydrogen peroxide (H₂O₂), by means of which a specified amount of hydrogen peroxide (H₂O₂) can be fed to the hydrogen sulphide absorption column (K02), and
**in that** the gas purification system (K01) has a storage and metering device (B02, D01) for sulphuric acid (H₂SO₄) taken off from the hydrogen sulphide absorption column (K01), by means of which a specified amount of sulphuric acid (H₂SO₄) can be fed to the ammonia absorption column (K01).

9. Gas purification system according to Claim 8, **characterized in that** the storage and metering devices have in each case at least one storage container (D02, B02) and at least one metering pump (D01).

10. Gas purification system according to Claim 8 or Claim 9, **characterized in that** each absorption column (K01, K02) has a circulation pump (P01, P02) between bottom and top, by means of which the respective wash solution can be pumped from the bottom to the top, where the wash solution can be added to a bed of packings through which the impure biogas stream can flow countercurrently.

11. Gas purification system according to any of Claims 8 to 10, **characterized in that** an adsorber (F01) is connected downstream of the hydrogen sulphide absorption column (K02).

12. Gas purification system according to Claim 11, **characterized in that** the adsorber (F01) has active carbon as an adsorbent.

13. Gas purification system according to any of Claims 8 to 12, **characterized**
**in that** the hydrogen sulphide absorption column (K02) is connected downstream of the ammonia absorption column (K01), and
**in that** a storage container (B01) for ammonium sulphate ((NH₄)₂SO₄) is connected downstream of the ammonia absorption column (K01).

14. Gas purification system according to any of Claims 8 to 13, **characterized in that** the gas purification system has a storage and metering device (D03) for sodium hydroxide (NaOH), by means of which a specified amount of sodium hydroxide (NaOH) can be fed, as a function of the pH to be set in the hydrogen sulphide absorption column (K02), to the hydrogen peroxide (H₂O₂) to be fed to the hydrogen sulphide absorption column (K02).

15. Gas purification system according to Claim 14, **characterized in that** the specified amount of sodium hydroxide (NaOH) can be fed in in the region of a bottom and top pipeline between bottom and top of the hydrogen sulphide absorption column (K02) or in the region of an H₂O₂ feed line to the bottom and top pipeline, in particular by means of a metering device.

## Revendications

1. Procédé de purification de biogaz produit par une installation de production de biogaz, dans laquelle de l'hydrogène sulfuré (H₂S) et de l'ammoniac (NH₃) sont éliminés au moins en partie du biogaz non purifié,
**caractérisé en ce**
**que** l'on ajoute du peroxyde d'hydrogène (H₂O₂) au biogaz, dans une étape de purification de l'hydrogène sulfuré (K02), et
en ce que l'acide sulfurique (H₂SO₄) formé par la réaction du peroxyde d'hydrogène (H₂O₂) et de l'hydrogène sulfuré (H₂S) à l'étape de purification de l'hydrogène sulfuré (K02) est acheminé au moins partiellement à une étape de purification de l'ammoniac (K01) dans laquelle du sulfate d'ammonium est formé par la réaction de l'ammoniac (NH₃) avec l'acide sulfurique (H₂SO₄).

2. Procédé selon la revendication 1, **caractérisé en ce que,** en aval de l'étape de purification de l'hydrogène sulfuré (K02) est réalisée une étape d'adsorption (F01) en tant que filtre de protection de sorte que le biogaz soit purifié à deux niveaux au-dessus et au dessous d'un seuil d'hydrogène sulfuré prédéterminé.

3. Procédé selon la revendication 2, **caractérisé en ce**
**que** le biogaz est pré-purifié à l'étape de purification de l'hydrogène sulfuré (K01) à une concentration d'hydrogène sulfuré de 40 à 60 mg/Nm³, de préférence, de 50 mg/Nm³, et
en ce que le biogaz ainsi pré-purifié est ensuite purifié à l'étape d'adsorption (F01) à une concentration d'hydrogène sulfuré inférieure ou égale à 5 mg/Nm3, de préférence, inférieure ou égale à 3 mg/Nm³.

4. Procédé selon l'une des revendications 1 à 3, **caractérisé en ce que** l'élimination de l'ammoniac est réalisée, à l'étape de purification de l'ammoniac (K01) à un pH de la solution de lavage présentant l'acide sulfurique (H₂SO₄) de 2 à 4.

5. Procédé selon l'une des revendications 1 à 4, **caractérisé en ce que** la quantité de dosage de l'acide sulfurique (H₂SO₄) est prédéterminée de telle sorte que la teneur en ammoniac dans le biogaz après l'étape de purification de l'ammoniac (K01) soit inférieure ou égale à 1 mg/Nm³.

6. Procédé selon l'une des revendications 1 à 5, **caractérisé en ce que** l'étape de purification de l'hydrogène sulfuré (K02) est effectuée après l'étape de purification de l'ammoniac (K01).

7. Procédé selon l'une des revendications 1 à 6, **caractérisé en ce que** l'on ajoute ou injecte au peroxyde d'hydrogène (H₂O₂), une quantité de soude caustique (NaOH) telle que le pH de l'étape de purification de l'hydrogène sulfuré (K02) présente une valeur prédéterminée, en particulier, un pH entre 7 et 10, de manière préférée entre toutes, entre 7 et 8,5.

8. Installation de purification de gaz pour purifier du biogaz d'une installation de production de biogaz, en particulier selon un procédé selon l'une des revendications 1 à 7,
avec une colonne d'absorption de l'hydrogène sulfuré pour l'élimination au moins partielle d'hydrogène sulfuré (H₂S) d'un biogaz non purifié et avec une colonne d'absorption d'ammoniac pour l'élimination au moins partielle de l'ammoniac (NH₃) du biogaz non purifié,
**caractérisée en ce**
**que** l'installation de purification de gaz présente un dispositif d'accumulation et de dosage (D02) du peroxyde d'hydrogène (H₂O₂) au moyen duquel on peut acheminer à la colonne d'absorption de l'hydrogène sulfuré (K02) une quantité prédéterminée de peroxyde d'hydrogène (H₂O₂), et
en ce que l'installation de purification de gaz (K01) présente un dispositif d'accumulation et de dosage (B02, D01) de l'acide sulfurique (H₂SO₄) retiré de la colonne d'absorption d'hydrogène sulfuré au moyen duquel on peut acheminer une quantité prédéterminée d'acide sulfurique (H₂SO₄) à la colonne d'absorption de l'ammoniac (K01).

9. Installation de purification de gaz selon la revendication 8, **caractérisée en ce que** les dispositifs d'accumulation et de dosage présentent respectivement un réservoir de stockage (D02, B02) et au moins une pompe doseuse (D01).

10. Installation de purification de gaz selon la revendication 8 ou la revendication 9, **caractérisée en ce que** chaque colonne d'absorption (K01, K02) présente une pompe en circuit (P01, P02) entre le fond et la tête, au moyen de laquelle la solution de lavage respective du fond peut être aspirée dans la tête où la solution de lavage peut être laissée sur un garnissage en vrac qui peut être traversé à contre courant par le courant de biogaz.

11. Installation de purification de gaz selon l'une des revendications 8 à 10, **caractérisée en ce qu'**après la colonne d'absorption d'hydrogène sulfuré (K02) est monté un adsorbeur (F01).

12. Installation de purification de gaz selon la revendication 11, **caractérisée en ce que** l'adsorbeur (F01) présente du charbon actif comme agent adsorbant.

13. Installation de purification de gaz selon l'une des revendications 8 à 12, **caractérisée en ce**
**que** la colonne d'absorption de l'hydrogène sulfuré (K02) est montée après la colonne d'absorption d'ammoniac (K01) et qu'après la colonne d'absorption d'ammoniac (K01) est monté un conteneur de stockage (B01) du sulfate d'ammonium ((NH₄)₂SO₄).

14. Installation de purification de gaz selon l'une des revendications 8 à 13, **caractérisée en ce que** l'installation de purification de gaz présente un dispositif d'accumulation et de dosage (D03) de soude caustique (NaOH), au moyen duquel on peut acheminer une quantité prédéterminée de soude caustique (NaOH) au peroxyde d'hydrogène (H₂O₂) conduisant à la colonne d'absorption d'hydrogène sulfuré (K02), indépendamment du pH à ajuster dans la colonne d'absorption d'hydrogène sulfuré (K02).

15. Installation de purification de gaz selon la revendication 14, **caractérisée en ce que** la quantité prédéterminée de soude caustique (NaOH) peut être pulvérisée dans la zone d'une conduite de fond et de tête entre le fond et la tête de la colonne d'absorption d'hydrogène sulfuré (K02) ou dans la zone d'une conduite d'acheminement de H₂O₂ à la conduite de fond et de tête, en particulier par un dispositif à buses.
